Europäisches Patentamt

European Patent Office

Office européen des brevets

(19)

(11) Publication number: **0 394 971**
**A1**

## EUROPEAN PATENT APPLICATION

(21) Application number: **90107783.4**

(22) Date of filing: **24.04.90**

(51) Int. Cl.5: **A61K 31/725, C08B 37/10**

(30) Priority: **24.04.89 US 342065**

(43) Date of publication of application:
**31.10.90 Bulletin 90/44**

(84) Designated Contracting States:
**AT BE CH DE DK ES FR GB GR IT LI LU NL SE**

(71) Applicant: **KabiVitrum AB**
**Lindhagensgatan 133**
**S-112 87 Stockholm(SE)**

Applicant: **PRESIDENT AND FELLOWS OF**
**HARVARD COLLEGE**
**University Place, 124 Mount Auburn**
**Cambridge, Massachusetts 02138(US)**

(72) Inventor: **Bergendahl, Karin Helena Louise**
**Nora-Torg 13**
**S-18234 Danderyd(SE)**
Inventor: **Johansson Rolf Arne**
**Daggstigen 8 B**
**S - 14138 Huddinge(SE)**
Inventor: **Mattson, Jan Christer**
**Rinkebyvagen 2**
**S - 17237 Sundbyberg(SE)**
Inventor: **Svan, Carl Magnus Erik**
**Leksandsvagen 12**
**S - 191 71 Sollentuna(SE)**
Inventor: **Folkman, Moses Judah**
**18 Chatham Circle**
**Brookline,Massachusetts 02146(US)**
Inventor: **Sudhalter, Judith**
**650 Huntington Avenue No. 7C Boston**
**Massachusetts 02115(US)**
Inventor: **D'Amore, Patricia**
**50 Jane Road, Newton**
**Massachusetts 02159(US)**

(74) Representative: **Burford, Anthony Frederick**
**W.H. Beck, Greener & Co. 7 Stone Buildings**
**Lincoln's Inn**
**London WC2A 3SZ(GB)**

(54) **Oligosaccharide-containing inhibitors of endothelial cell growth and angiogenesis.**

(57) Novel fractions comprising oligosaccharides having 6 to 8 saccharide units and molecular weights between 1,000 and 2,000 and a sulfur content of less than 9 weight percent, are obtained by depolymerizing heparin or heparan sulfate and fractionating the digest by size and charge. These fractions inhibit capillary endothelial cell proliferation in vitro and are useful in the in vivo inhibition of angiogenesis.

## OLIGOSACCHARIDE-CONTAINING INHIBITORS OF ENDOTHELIAL CELL GROWTH AND ANGIOGENESIS

The present invention relates to novel inhibitors of endothelial cell growth and angiogenesis which constitute saccharide mixtures which are derived from heparin or heparan sulfate, particularly such saccharide mixtures that contain as a major component oligosaccharides containing 6 to 8 saccharide units and of low sulfate content; to a process for obtaining such saccharide mixtures; and to the use of such fractions in therapy.

Angiogenesis, the growth of new capillary blood vessels, is important in normal processes such as development of the embryo, formation of the corpus luteum and wound healing. It is also a component in pathologic processes such as chronic inflammation, certain immune responses and neoplasia. Angiogenesis is also a property of most solid tumours and is necessary for their growth.

Reduced angiogenesis is desirable in treatment of inter alia tumours, especially solid tumours, neovascular diseases of the eye such as retrolental fibroplasia, diabetic retinopathy and neovascular glaucoma, in the treatment of osteoporosis, psoriasis and arthritis and in the prevention of metastasis.

The growth of new capillary blood vessels starts from the endothelial cells that line the blood vessels and inhibition of growth of endothelial cells can be considered to be a model of angiogenesis.

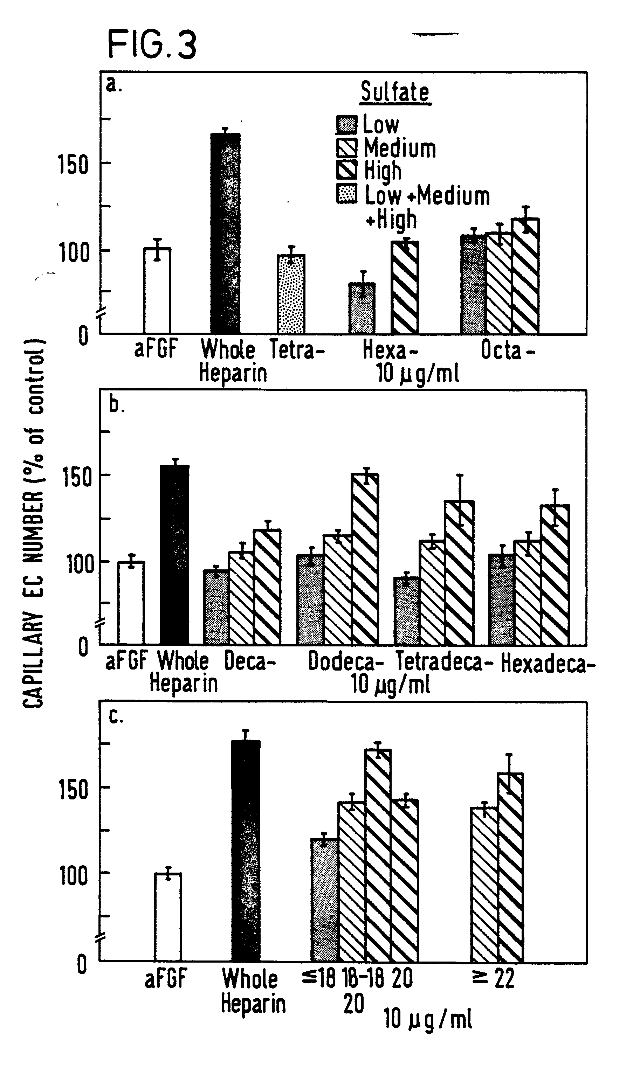

It has been found by Thorton et al. (Science 1983, 222, 623-625) that heparin potentiates the mitogenic activity of acidic fibroblast growth factor (aFGF) on endothelial cells in vitro. Recently this finding was extended in a study by Sudhalter et al. (J. Cell Biol 1988, 107, 874a). Sudhalter et al. depolymerized porcine intestinal mucosa heparin with nitrous acid. This resulted in a heterogeneous mixture of fragments containing saccharides ranging from disaccharides to polysaccharides of about 40 saccharide units in length. This mixture of heparin fragments was further fractionated by ion exchange chromatography and gel permeation chromatography to obtain size-homogenous oligosaccharides with differing degrees of sulfation. Assay of these saccharides on endothelial cell proliferation in the absence of added aFGF revealed that all of the saccharides tested were capable of inhibiting endothelial cell proliferation by an average of 25% over a 3 day assay. Assay in the presence of a suboptimal concentration of aFGF indicated that a minimum length of 10-12 saccharide units was necessary for potentiation of the aFGF induced cell growth. To obtain a stimulatory effect equal to heparin a high sulfate dodecasaccharide (12 sugar units) had to be used. It was also shown that a high sulfate hexasaccharide had no effect on endothelial cell growth in the presence of aFGF (see also Figure 3). Said hexasaccharide also showed the same small inhibitory effect that the other heparin derived oligosaccharides, as well as heparin itself, had on endothelial growth in the absence of aFGF.

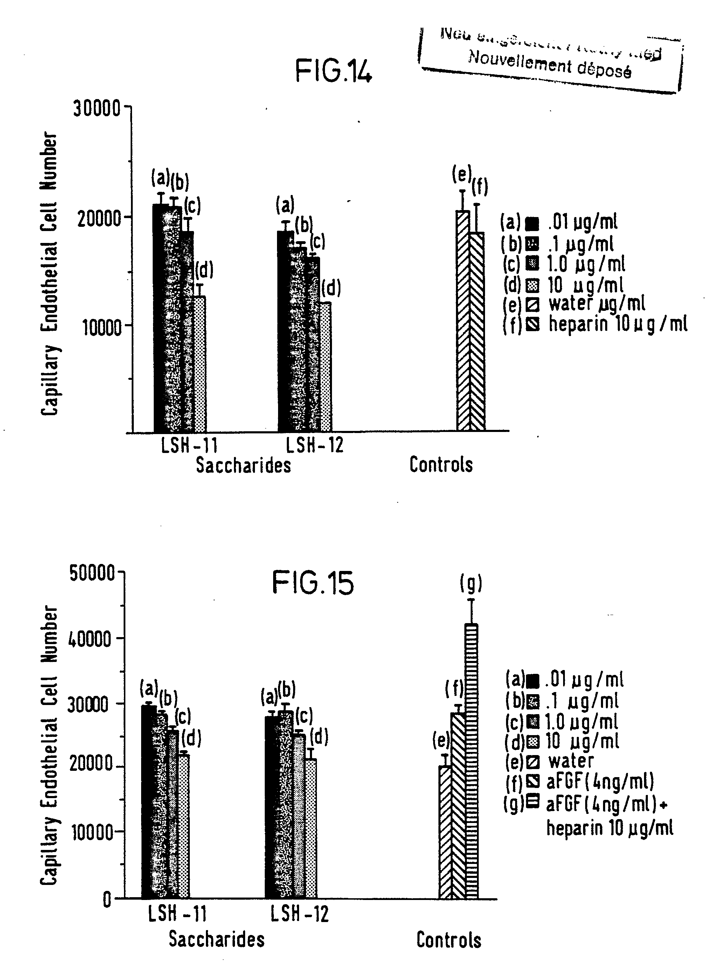

In EP-A-0244298 a hexasaccharide preparation is described which was obtained by affinity chromatography on a Sepharose column containing aFGF. This hexasaccharide preparation which is highly sulfated (see also Lormeau et al. 1987 Angiogenesis, Mechanisms and Pathobiology Current Communications in Molecular Biology, Cold Spring Harbor Laboratory p. 43) is characterized by a disaccharide repeating structure -(G-H)$_n$-G- or -H-(G-H)$_n$ where G is L-iduronic acid 2-0-sulfate and H is D-glucosamine N-sulfate-6-0-sulfate. This hexasaccharide preparation was shown to have a weak stimulatory effect on endothelial cells from the umbilical vein in the presence of aFGF (EP-A-0244298; Fig. 14 C). On other cell types such as rat and calf vascular aortic smooth muscle cells and rat cervical epithelial cells, hexasaccharides from heparin showed some antiproliferative properties. On all these other cells however the inhibition of growth was associated with highly sulfated hexasaccharides (Wright et al., J Biol Chem 1989, 264, 1534-1542) which have a sulfur content substantially greater than 9 weight percent.

European patent specification EP 0114589 (Harvard College) discloses a composition suitable for oral or subcutaneous administration, for inhibition of angiogenesis in mammals, in which the active agents consist essentially of 1) heparin or a heparin fragment which is a hexasaccharide or larger and 2) cortisone or hydrocortisone or the 11-alpha isomer of hydrocortisone is claimed. Such a two component mixture causes inhibition of angiogenesis in mammals and tumour masses in mammals are caused to regress and metastasis is prevented (Folkman et al., Science 1983, 221, 719-725). The mixture is also expected to be effective in treating other diseases involving neovascularization such as neovascular diseases of the eye, psoriasis and arthritis.

EP-A-0302034 (Kabi Vitrum AB) discloses novel heparin derivatives consisting of copper complexes of heparin fragments including hexasaccharide fragments together with an angiostatic component, especially a so-called angiostatic steroid component, for use in therapy.

It would be particularly useful in therapy to have access to heparin derived preparations which inhibit angiogenesis without requiring the presence of steroids. It would also be useful is such preparations could be delivered orally, as well as by topical or parenteral administration.

EP 0 394 971 A1

It has now unexpectedly been found that fractions containing low sulfate oligosaccharides having 6 to 8 saccharide units and derived from heparin or heparan sulfate, in contrast to the corresponding high sulfate hexasaccharide fractions, described above, show a markedly enhanced inhibition of endothelial cell growth and also show markedly anti-angiogenic properties particularly without additional angiostatic components such as angiostatic steroids.

Accordingly, the present invention provides fractions derived from heparin or heparan sulfate, said fractions comprising low molecular weight oligosaccharides having a molecular weight between 1,000 and 2,000, characterized in that the majority of said low molecular weight oligosaccharides are low sulfate oligosaccharides having a sulfur content of less than 9 weight percent, whereby the said fractions inhibit capillary endothelial cell proliferation or angiogenesis and do not have any significant anticoagulant activity.

Usually, said low sulfate oligosaccharides will have a molecular weight between 1,000 and 1,800, and preferably between 1,000 and 1,600. It is preferred that the sulfur content of said low sulfate oligosaccharides is less than 7 weight percent and preferably less than 4 weight percent.

Preferably, the fractions consist essentially of said low molecular weight oligosaccharides, and preferably they consist essentially of said low sulfate oligosaccharides.

It is believed that the low sulfate oligosaccharides usually comprise a uronic acid residue, a glucosamine residue and a galactose residue. These oligosaccharides may include glucuronic acid residues and may be essentially free of iduronic acid residues. The oligosaccharides may also include xylose residues. In particular, it is believed that the fractions usually have a terminal xylose residue connected to a galactose residue which in turn is connected to a further galactose residue. Said second galactose residue is believed usually to be connected to a uronic acid residue, attached via a glucosamine residue to a second uronic acid residue. Said glucosamine-uronic acid disaccharide unit can be repeated to form an octasaccharide. Preferably, any glucosamine residues are N-acylated, and may optionally carry a sulfate group in the 6-position.

It is believed also that the low sulfate oligosaccharides consist essentially of oligosaccharides having 6 to 8 saccharide units.

Preferably, the fractions are capable of inhibiting angiogenesis in chicken egg chorioallantoic membranes when administered in a dose between 1 $\mu$g and 50 $\mu$g.

The present invention also provides fractions for use in the inhibition of angiogenesis; the use of the fractions in the manufacture of a medicament for the inhibition of angiogenesis; the inhibition of angiogenesis using the fractions; and pharmaceutical compositions comprising the fractions with a pharmaceutically acceptable carrier or diluent.

Suitably, the novel fractions of the present invention are isolated from heparins or heparan sulfates of bovine, porcine or other mammalian origin; low molecular weight heparins or heparan sulfates; fragments of heparins and of heparan sulfate; oligosaccharides from any of these products; size homogeneous oligosaccharides having 6 to 8 saccharide units; and other fractions containing heparin or heparan sulfate constituents, for example byproducts from heparin production.

As described above, the new fractions may contain disaccharide sequences consisting of an uronic acid residue and a glucosamine residue. The uronic acid residue is either glucuronic acid or iduronic acid which optionally can be substituted by a sulfate group in the 2-position. The most common uronic acid unit is glucoronic acid without any sulfate substituent. As described above, the glucosamine unit is preferably acetylated on the amino group and can optionally carry a sulfate group in the 6-position. However, one of the glucosamines of the oligosaccharide can alternatively be substituted by a sulfate group on the amino group or carry no substituent at all on the amino group. One of the galactose units may optionally carry a sulfate group and the xylose unit may optionally carry a phosphate group. An O-linked serine residue may optionally be attached to the oligosaccharide via the xylose unit.

The new fractions of the invention are prepared by partial or exhaustive depolymerization of heparin and/or heparan sulfate containing products by chemical or enzymatic methods. The chemical methods employed normally make use of a nitrite reagent, such as a salt of nitrous acid, preferably the sodium salt, or an organic nitrite in an aqueous solvent, or of an alkaline reagent such as sodium hydroxide in aqueous solvent or a quaternary ammonium hydroxide in an organic solvent. When using an alkaline reagent the heparin/heparan sulfate starting product preferably is esterified on the carboxylic groups. Enzymes such as heparinase, heparitinase or $\beta$-glucuronidases such as heparanase may also be used for depolymerization. After depolymerization the newly formed end groups may be reduced by sodium borohydride or by using hydrogen plus a metal catalyst or, in the case when anhydromannose is formed, oxidation can be used to form anhydromannaric acid terminal residues. The fractions thus formed are then subjected to ion exchange chromatography at low pressure or at elevated pressure employing a weak or strong basic anion exchange resin and/or fractionation by gel permeation chromatography at low pressure or at elevated pressure in

3

order to obtain fractions that contain low sulfate oligosaccharides having 6 to 8 saccharide units as major components. Further fractionation by ion exchange chromatography and/or gel permeation chromatography may be carried out in order to obtain fractions which are homogeneous with respect to size and contain decreasing amounts of sulfate groups. Examples of anion exchange resins include DE-32 (Whatman) or Amberlite (Trade Mark) IRA-47, 401, 904, Dowex (Trade Mark) 1x1, 1x2, DEAE-Sepharose (Trade Mark), Q-Sepharose (Trade Mark) and Mono Q (Trade Mark) (Pharmacia). Separation according to degree of sulfation (charge density) may also be carried out by forming a complex with a quaternary ammonium ion such as cetyltrimethylammonium, cetylpyridinium, Hyamin (Trade Mark), benzalkonium, or other quaternary ammonium ions and then precipitating the most sulfated (highly charged) fractions with an organic solvent such as ethanol, methanol or acetone and recovering low sulfate (less highly charged) fractions from the mother liquor. An enrichment of low sulfate containing fractions can also be accomplished by extracting the low sulfated portion from a heterogeneous mixture by an aqueous alcohol solvent extraction (containing less than 45% water) and then recovering the dissolved low sulfate oligosaccharides by evaporation of the solvent.

In clinical practice, administration of the novel fractions of the present invention will be essentially as for heparin and low molecular weight heparin. Thus, topical administration and parenteral administration, especially by intravenous, intramuscular or subcutaneous injections, can be used. Local administration, e.g. in the eye or to a tumour, may be used when desired. Advantageously, however, due to their low molecular weight and low sulfate content (with associated reduced charge) the fractions may also be administered orally.

For local administration the amount administered usually will be 0.1-10 mg/day. For parenteral administration 10-1000 mg/day usually will be used and for oral application up to 5, or even 10, g/day may be needed.

The new low sulfate oligosaccharide fractions derived from heparin and/or heparan sulfate are characterized by the following features:

(a) they show enhanced inhibition of angiogenesis;

(b) they need no additional angiostatic steroid for said inhibition to take place;

(c) they inhibit strongly the growth of capillary endothelial cells;

(d) they inhibit strongly the growth of capillary endothelial cells also in the presence of acidic Fibroblast Growth Factor (aFGF); and

(e) they do not have any significant anticoagulant activity (ie. greater than 5 U/mg) as measured by an anti-FXa assay.

Presently preferred embodiments of the invention are disclosed hereinafter with reference to non-limiting Examples and accompanying Figures. In the Figures:

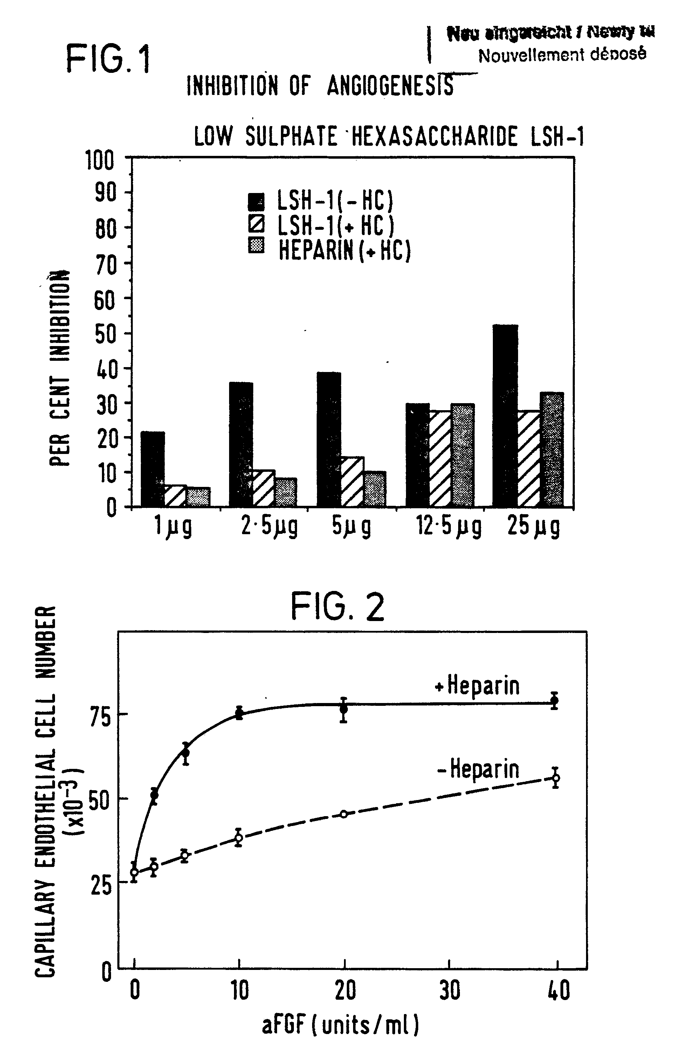

Figure 1 shows the inhibition of angiogenesis by a low sulfate oligosaccharide of the invention LSH-1 (believed to be predominantly hexasaccharide) compared to the heparin it was prepared from. The assay procedure was as described in the Biological Tests hereinafter. Results from 20-50 chicken eggs are included in each column. HC means hydrocortisone.

Figure 2 shows the effect of heparin on the mitogenic effect of aFGF on capillary endothelial cells. Cell proliferation assays were conducted as described in the Biological Tests hereinafter and challenged with varying doses of aFGF (2.5-40 3T3 units) in the presence and absence of heparin (10 µg/ml). Following a 3 day incubation, the cells were trypsinized and counted electronically.

Figure 3 shows the effect of heparin-derived oligosaccharides of varying sizes and degrees of sulfation on the mitogenic effect of aFGF on capillary endothelial cells. Cell proliferation assays were conducted as described in the Biological Tests hereinafter. Controls consisted of cells grown in the presence of aFGF (2 units/ml) alone (empty bar) and aFGF (2 units/ml) plus whole heparin (10 µg/ml) (solid bar). Only the low sulfate oligosaccharide of the invention (LSH-1) inhibited the aFGF stimulated cell proliferation.

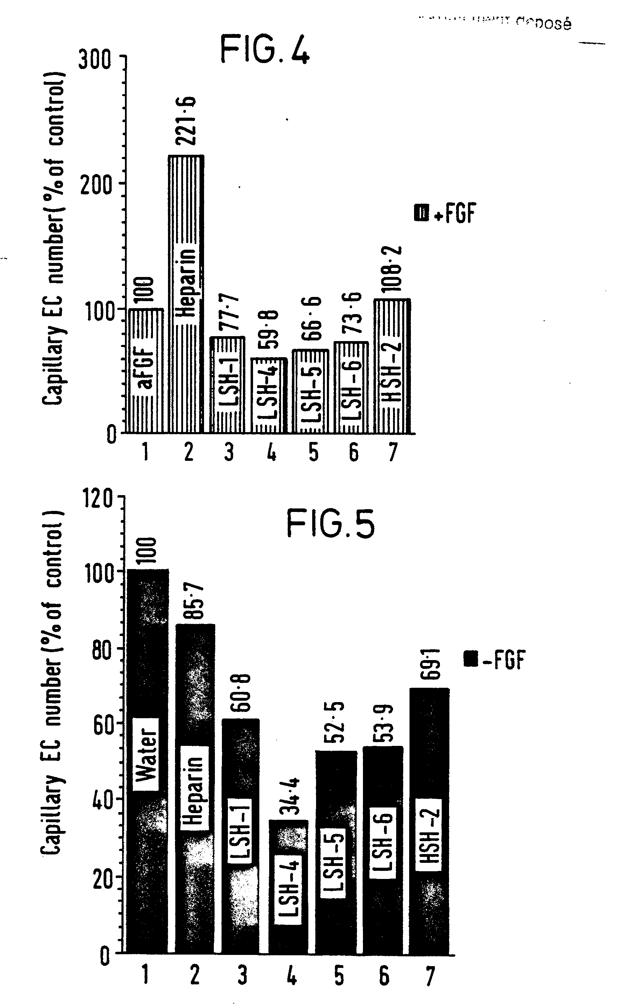

Figure 4 shows the effect of heparin-derived oligosaccharides of varying sizes and degrees of sulfation on the mitogenic effect of aFGF on capillary endothelial cells. Cell proliferation assays were conducted as described in the Biological Tests hereinafter. Controls consisted of cells grown in the presence of aFGF (2 U/ml) alone and aFGF (human recombinant, 2 ng/ml) plus standard heparin (10 µg/ml). Three of the four subfractions from low sulfate oligosaccharide LSH-1 showed enhanced inhibition of cell proliferation, whereas the fourth subfraction (HSH-2) which is of higher sulfate content than the starting fraction LSH-1 showed less inhibition than LSH-1.

Figure 5 shows the effect of heparin-derived oligosaccharides of varying degrees of sulfation on capillary endothelial cells. Cell proliferation assays were conducted as described in the Biological Tests hereinafter. Controls consisted of cells grown in the presence of water (the vehicle for heparin and the saccharides) and heparin alone. All the low sulfated oligosaccharides of the invention LSH-1, LSH-4, LSH-5

4

and LSH-6 were good inhibitors, whereas the oligosaccharide of higher sulfate content (HSH-2) was less inhibitory.

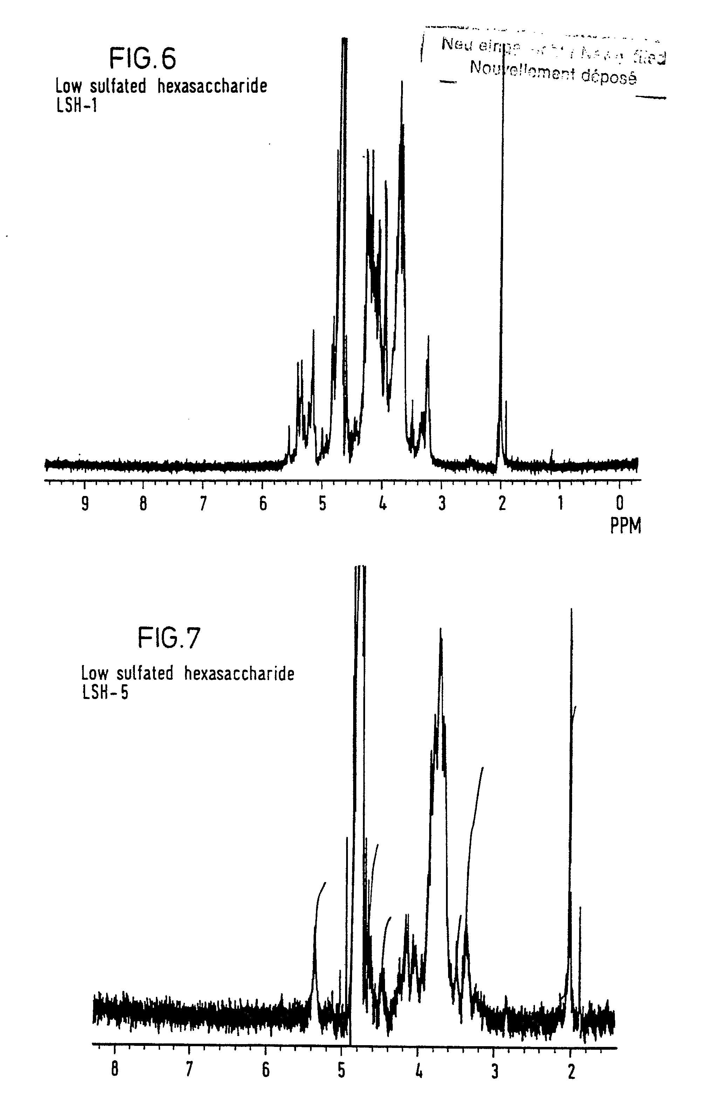

Figure 6 shows the 'H-NMR spectrum of the low sulfate oligosaccharide of the invention LSH-1.

Figure 7 shows the 'H-NMR spectrum of the low sulfate oligosaccharide of the invention LSH-5. NMR-spectra were recorded in $D_2O$ on a JEOL GX-400 instrument (400 MHZ 'H) at 25°C using water at 4.77 ppm as reference.

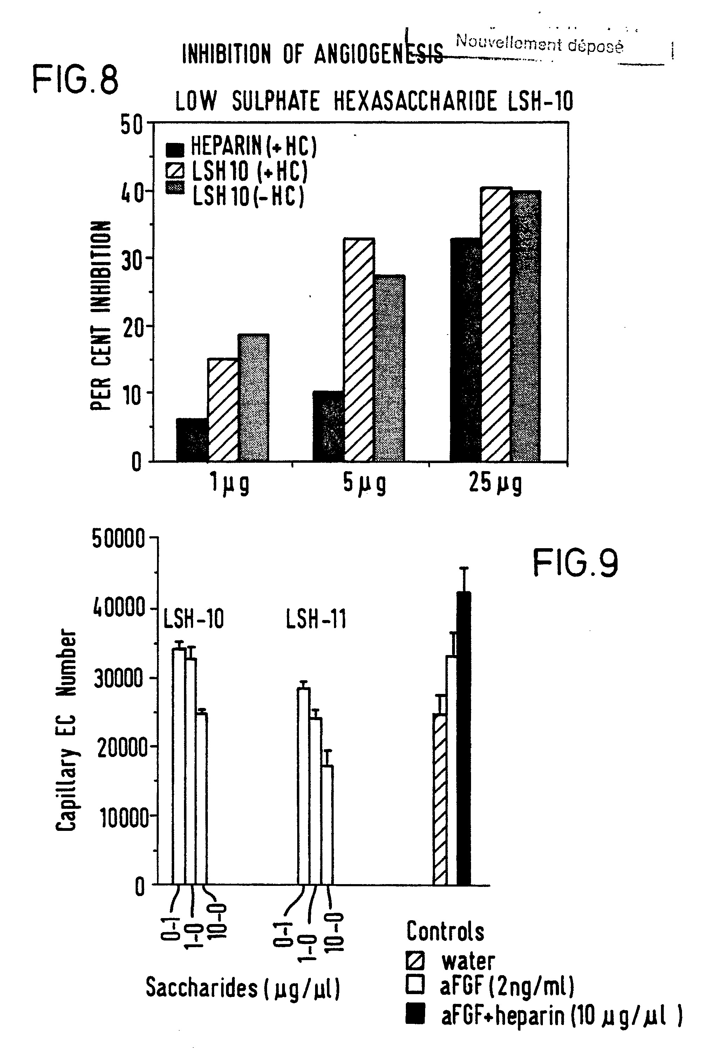

Figure 8 shows the inhibition of angiogenesis of low sulfate oligosaccharide of the invention LSH-10 (believed to be predominantly hexasaccharide) in the presence (+ HC) and absence (- HC) of hydrocortisone compared to the heparin (in the presence of hydrocortisone; + HC) it was prepared from. The assay procedure was as described in the Biological Tests hereinafter. Results from 20-50 eggs are included in each column.

Figure 9 shows the dose response effect of low sulfate oligosaccharides of the invention LSH-10 (believed to be a hexasaccharide) and LSH-11 (believed to be an octasaccharide) on the mitogenic effect of aFGF on capillary endothelial cells. Cell proliferation assays were conducted as described in the Biological Tests hereinafter. Controls consisted of cells grown in the presence of water (the vehicle for heparin and the saccharides) (cross-hatched bar), in the presence of aFGF (2 ng/ml) alone (empty bar) and aFGF (2 ng/ml) plus whole standard heparin (10 μg/ml) (solid bar). The increased inhibition of aFGF stimulated cell proliferation by increasing doses of LSH-10 and LSH-11 is clearly seen.

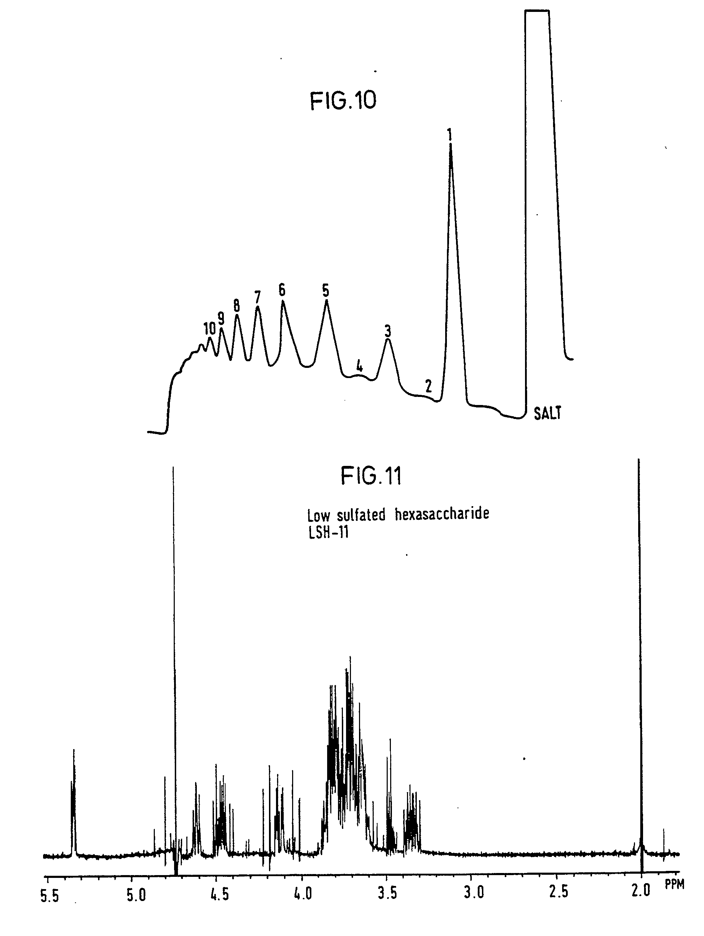

Figure 10 shows the results of gel permeation chromatography of depolymerized herapin on a Biogel-P6 column (1800 x 50 mm) with 0.25M sodium chloride as an eluent at 3 cm/h. Chromatography was monitored by a refractive index detector and fractions were pooled which corresponded to peaks in the chromatogram. 5 g of salt containing a mixture of low and medium sulfated oligosaccharides from nitrous acid depolymerized heparin was applied to the column in each run.

Figure 11 shows the 'H-NMR spectrum of low sulfate oligosaccharide of the invention LSH-11 (believed to be octasaccharide). This NMR spectrum was recorded in $D_2O$ on a JEOL GX-400 instrument (400 MHZ 'H) at 25°C using water at 4.73 ppm as reference.

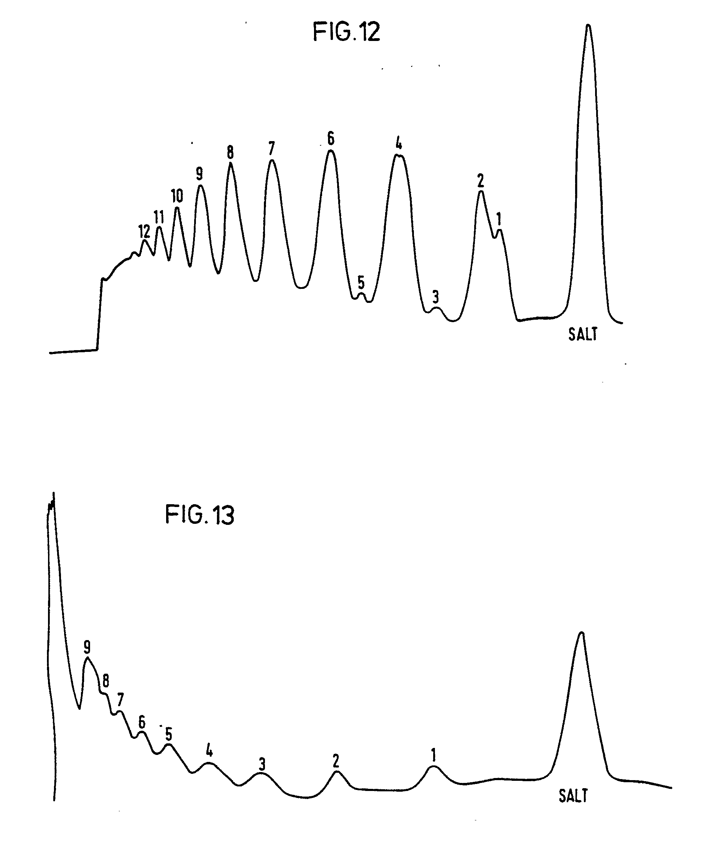

Figure 12 shows the results of gel permeation chromatography of depolymerized heparin on a Biogel P-6 column (1800 x 50 mm) using sodium chloride as an eluent at 3 cm/h. Chromatography was monitored by a refractive index detector. Depolymerized heparin (8 g) was dissolved in sodium chloride (0.25M) and added to the column.

Figure 13 shows the results of gel permeation chromatography of depolymerized heparan sulfate on a Biogel-P6 column (1800 x 50 mm) with 0.25M sodium chloride as an eluent at 3 cm/h. Depolymerized heparan sulfate 0.5 g was dissolved in sodium chloride (0.25M) and applied to the column. The elution was monitored using a refractive index detector.

Figure 14 shows the dose response effect of low sulfate oligosaccharides of the invention LSH-11 and LSH-12 (believed to be a hexasaccharide) on the effect of proliferation of capillary endothelial cells in the absence of aFGF. Cell proliferation assays were conducted as described in the Biological Tests hereinafter. Controls consisted of cells grown in the presence of water (the vehicle for heparin and the saccharides) (empty bar) and whole standard heparin (solid bar) 10 μg/ml. The increased inhibition of cell proliferation by increasing doses of LSH-11 and LSH-12 is clearly seen.

Figure 15 shows the dose response effect of low sulfate oligosaccharides of the invention LSH-11 and LSH-12 on the effect of proliferation of capillary endothelial cells in the presence of aFGF. Cell proliferation assays were conducted as described in the Biological Tests hereinafter. Controls consisted of cells grown in the presence of water (the vehicle for heparin and the saccharides) (empty bar) and aFGF (4 ng/ml) plus standard heparin (10 μg/ml) (cross-hatched bar). The increased inhibition of aFGF stimulated cell proliferation by increasing doses of LSH-11 and LSH-12 is clearly seen.

Example 1

Heparin fragments were obtained by partial deaminative cleavage of heparin from porcine intestinal mucosa using nitrous acid essentially as previously described by Thunberg et al. (FEBS letter 1980. 117, 203-206) followed by reduction by sodium borohydride according to Horton and Philips (Carbohydr Res 1973, 30, 367 - 374). The solution of the heparin fragments was subjected to ion exchange chromatography on DEAE-Sepharose using sodium chloride as an eluent. Three fractions were collected: a low sulfate fraction at 0.15 M sodium chloride, sulfur/carbon atomic ratio (by elemental analysis) 0.14; a medium sulfate fraction at 0.6 M sodium chloride, sulfur/carbon atomic ratio 0.19; and a high sulfate fraction, sulfur/carbon atomic ratio 0.21 at 1.5 M sodium chloride. According to high performance gel permeation chromatography (HPGPC), these fractions contained saccharides ranging in size from tetra- or hexasaccharides to those the size of the original heparin (average $M_r$ = 13,500). Each of the low sulfate, medium sulfate and high sulfate fractions was further separated into size-homogeneous, even-numbered oligosaccharides by gel permeation chromatography on Sephadex G-50 Superfine according to the methods of Lane et al. (Biochem. J. 1984, 218, 735), giving the fractions shown in Table 1. Tetrasaccharides were only obtained from the low sulfate fraction. Oligosaccharides believed to be hexasaccharides were obtained from the low, medium and high sulfated fractions. As the sulfur to carbon ratio was high in hexasaccharides from both the medium and the high sulfate fractions, these hexasaccharide fractions were pooled (results shown in 'high' sulfate column, Table I). All fractions were analyzed for C, N and S at Mikro Kemi AB, Uppsala, Sweden. HPGPC was carried out using two gel permeation TSK G3000SW columns connected in series. The mobile phase was 0.2 M sodium acetate containing 0.05% v/v acetic acid and 0.02% w/v sodium azide. The flow rate was 0.5 ml/min. The sample was dissolved in the eluent and Blue dextran and sodium azide were added as markers. Detection was performed with an RI-detector. Chart speed was 2 mm/min. Peaks in the chromatogram were assigned by comparison to typically sulfated (ie. about 2 sulfate groups per disaccharide unit) disaccharides, tetrasaccharides and hexasaccharides, which had been characterized by mass spectroscopy.

A fraction believed to be predominantly low sulfate hexasaccharide (LSH-1) contained 8.6% S and a fraction believed to be predominantly high sulfate hexasaccharide (HSH-1) contained 11.9% S compared to the starting heparin which contained 10.7% S. Further characterization of LSH-1 can be deduced from the 'H NMR spectrum (see Figure 6). Using 25 µg LSH-1 per egg together with 50 µg hydrocortisone LSH-1 showed 28% inhibition of angiogenesis and HSH-1 showed only 3% inhibition of angiogenesis. For the activity of LSH-1 in absence of hydrocortisone see Figure 1. For the marked inhibition of endothelial cell proliferation in the presence of aFGF see Figure 3.

TABLE 1

Properties of oligosaccharides obtained from nitrous acid depolymerized heparin[1] by ion exchange chromatography and gel permeation chromatography.

| Number of monosaccharides in oligo-saccharide | Low sulfate | Medium sulfate | High sulfate |
|---|---|---|---|
| | $\dfrac{Sulfur[2]}{carbon}$ | $\dfrac{Sulfur}{carbon}$ | $\dfrac{Sulfur}{carbon}$ |
| 4 | 0.16 | | |
| 6 | 0.14 | | 0.22 |
| 8 | 0.13 | 0.18 | 0.20 |
| 10 | 0.11 | 0.19 | 0.21 |
| 12 | 0.11 | 0.17 | 0.21 |
| 14 | 0.11 | 0.19 | 0.22 |
| 16 | 0.11 | 0.17 | 0.22 |
| 18 | 3) | | 0.22 |
| 18-20 | | 0.17 | |
| 20 | | | 0.21 |
| 22[4] | | 0.17 | 0.20 |

1) The ratio sulfur/carbon atoms of heparin from porcine intestinal mucosa determined for 11 different heparins was 0.18 ± 0.01.

2) Ratio of sulfur/carbon atoms determined by elemental analysis.

3) Mixture of polysaccharides consisting of saccharides from 18 monosaccharides to about the average size of the original heparin sulfur/carbon ratio not determined.

4) Mixture of polysaccharides consisting of mono-saccharides from 22 to about the average size of the original heparin.

Example 2

A heparin fragment was obtained by exhaustive nitrous acid depolymerization and chromatography on

7

DEAE-Sepharose. After purification on Sephadex G-10 a fraction consisting largely of oligosaccharides believed to be predominantly hexasaccharides was obtained. Accordingly to elemental analysis nitrogen was 1.5% and sulfur was 3.2%. The inhibition of angiogenesis for LSH-2 at 25 μg was 50% (in the presence of hydrocortisone). The inhibition without hydrocortisone was higher.

Example 3

The low sulfate oligosaccharide (LSH-1 from Example 1) was further fractionated by ion exchange chromatography on the strong anion exchange resin using Mono Q in a 5/5 HR column (Pharmacia) and eluted with a sodium chloride gradient. The gradient was formed by two LKB 2150 pumps controlled by a LKB 2152 unit. The eluent was monitored by an ultraviolet detector (LKB 2151) set at 218 nm.

LSH-1 (49 mg) was dissolved in water (1 ml). 100 ul of this solution was injected to the column through a Rheodyne injector. Separation was obtained by isochratic elution at 1.00 ml/min for ten minutes with 60mM sodium chloride, and then a linear increase at 1.00 ml/min for 55 minutes up to 1.2 M sodium chloride. Finally the gradient was held at 1.2 M sodium chloride at 1.00 ml/min for 15 minutes. 1 ml fractions were collected, and material was pooled as follows:

| fraction #1 | 1-10 ml |
| fraction #2 | 11-26 ml |
| fraction #3 | 27-40 ml |
| fraction #4 | 41-75 ml |

These respective fractions were concentrated under vacuum and applied to a P2 column (78 x 26 mm) and eluted with water at 11.3 cm/h. The eluent was monitored by refractive index detection, and 15 minute fractions were collected. Carbohydrate containing fractions were pooled and lyophilized to give three low sulfate containing oligosaccharide fractions believed to be predominantly hexasaccharides (LSH-4, LSH-5 and LSH-6) and one relatively high sulfate containing fraction (HSH-2). For the marked inhibition of endothelial cell proliferation see Figures 4 and 5.

LSH-4 1.5 mg (from fraction #1) S less than 3%
LSH-5 4.8 mg (from fraction #2) S less than 3% 'H-NMR spectrum (Figure 7)
LSH-6 7.1 mg (from fraction #3) S = 6.0%
HSH-2 29.5 mg (from fraction #4) S = 10.5% N = 1.5%
From Figure 7 the very low content of N-sulfate groups and the absence of sulfated iduronic acid in LSH-5 can clearly be seen.

Example 4

Heparin from porcine intestinal mucosa was depolymerized by alkaline β-elimination of the heparin methylester according to EP-A-0302034. The product was applied to Sephadex G-50 and eluted with 0.2 M sodium chloride. One of the fractions collected had a molecular weight of 2000 (by gel filtration) after desalting on Sephadex G-10 and an anti-Xa activity of less than 5 U/mg ($A_{231}$ = 19). This product (25 mg) was applied to a strong ion exchange resin Mono Q 10/10 (Pharmacia). Fractions were eluted by a sodium chloride gradient from 0-1.5 M sodium chloride in 0.03 M tris HCl-buffer pH 7.5. The gradient was linear for 156 min. The flow was 4 ml/min. After desalting on Sephadex G-10 and Sephadex G-15 the oligosaccharides were pooled in three fractions.

LSH-7 10 mg S = 8.9
LSH-8 3.8 mg
HSH-3 9 mg S = 12.6

Example 5

Heparin from porcine intestinal mucosa was depolymerized as described in Example 1. Depolymerized herapin (8 g) was dissolved in sodium chloride (0.25 M) and subjected to gel permeation chromatography on Biogel P-6 (1800 x 50 mm) using sodium chloride 0.25 M as an eluent at 3 cm/h. Chromatography was monitored by a refractive index detector and fractions were pooled according to the peaks of Figure 12. Fractions were desalted on Bio-gel P6DG with water, using a refractive index detector and then lyophilized. Fractions 4 to 7 were further examined for the low sulfated oligosaccharides of the invention.

Fraction 5 (5 g) was applied to a column (450 x 50 mm) containing QAE A-25 ion exchange resin in chloride form and eluted with a gradient of 0.01-2.0 M sodium chloride using UV-detector at 214 nm. Low sulfate oligosaccharides which eluted with 0.01-0.6 M sodium chloride were pooled and desalted as above. 200 mg of these saccharides was subjected to high performance ion exchange chromatography on the strong anion exchange resin Mono Q (10/10 HR column) and eluted with sodium chloride. First isochratic elution at 1.0 ml/min. for 10 min. with 10m M sodium chloride and then a gradient to 2.0 M sodium chloride. The eluent was monitored by a UV-detector set at 218 nm. 1 ml fractions were collected and material pooled as follows:

| Fraction 1 | 1-10 ml |
| Fraction 2 | 11-30 ml |
| Fraction 3 | 31-70 ml |

After concentration, desalting as described above and lyophilization LSH-10 (9 mg) was obtained from Fraction 1. On sugar analysis the following monosaccharides were found: xylose 3.0%, galactose 12.1%, fucose 1.9% and glucosamine 14.8%. No galactosamine, mannosamine or mannose were found. Small amounts of anhydromannitol (0.41%) and serine (0.08 mol/mol glucosamine) were also found. According to elemental analysis P was 1.0% and S was 0.5%. NMR-spectra showed presence of N-acetyl-glucosamine and glucuronic acid and absence of non-sulfated and sulfated iduronic acid. A FAB mass spectrum run in a negative mode showed the highest peaks at M/Z 1194 and 1176. The inhibition of capillary endothelial cell proliferation is seen in Figure 9 and the inhibition of angiogenesis is seen in Figure 8.

Example 6

Heparin was depolymerized by nitrous acid as described in Example 1. The depolymerized heparin (40 g) was dissolved in water (150 ml) and subjected to ion exchange chromatography on a QAE Sephadex (A-25 (750 x 50 mm) column. Elution was performed with water to obtain a fraction containing low and medium sulfated oligosaccharides ranging from tetrasaccharides to about eicosasaccharides. (Fraction No. 1) and with 2M sodium chloride to obtain high sulfated oligosaccharides of similar sizes (Fraction 2). The eluent was monitored by a UV-detector at 214 nm. The water fraction (Fraction 1) was concentrated and 5 g of the solid residue was dissolved in water and applied to gel permeation chromatography on a Biogel P-6 (1800 x 50 mm) with 0.25 M sodium chloride as an eluent at 3 cm/h. Chromatography was monitored by a refractive index detector and fractions were pooled which corresponded to peaks in the chromatogram (see Figure 10). The respective fractions were concentrated and desalted by chromatography on Biogel P-6DG gel (220 x 50 mm column) with water at 3 cm/h as an eluent. Detection of carbohydrate containing material was performed by a refractive index detector. Fractions 3 to 7 (see Figure 10) were further examined for the low sulfated oligosaccharides of the invention.

Fraction 5 (200 mg) was applied to a QAE 25 column (750 x 50 mm) which was eluted with a sodium chloride gradient (0.01-2.0 M). Three fractions (5A-C) corresponding to the three peaks of the chromatogram were collected and desalted. The first fraction (5A) was subjected to preparative high performance ion exchange chromatography on the strong anion exchange resin Mono Q in a 16/10 HR column and eluted with a sodium chloride gradient. Isochratic elution at 4.0 ml/min. for 23 min. using 10mM sodium chloride, then a gradient to 2.0 M sodium chloride. The eluent was monitored by an ultra-violet detector at 218 nm. The major peak LSH-11 which eluted at about 25 min. was concentrated and desalted as above to give 7 mg LSH-11. LSH-11 was subject to sugar analysis by hydrolysis, derivatization and GLC-MS, showing glucosamine 14.4%, xylose 3.2% and galactose 11.8%. No fucose, mannose, mannosamine, galactosamine or anhydromannitol was found. According to elemental analysis P was 0.2% and S was 0.2%. NMR spectra indicate the presence of N-acetyl-glucosamine and glucuronic acid and absence of iduronic acid as well as 2-0-sulfated iduronic acid (see Figure 11). A FAB mass spectrum run in a negative mode showed the highest peaks at M/Z 1553 and a base peak at M/Z 1429. Peaks at M/Z 1451, 1473, 1487, 1509 and 1531 were seen also, indicating an oligosaccharide. The strong inhibitory activity of LSH-11 on capillary endothelial cell proliferation in the presence of acidic FGF is seen in Figure 9. The inhibition of capillary endothelial cell proliferation in absence of aFGF was also very high as seen in Figure 14.

Fraction No. 3 (90 mg) was subjected to preparative high performance ion exchange chromatography on Mono Q (16/10 HR) and eluted with a sodium chloride gradient. Separation was obtained by isochratic elution at 3.0 ml/min. for 30 min. using 10 mM sodium chloride and then a gradient to 2.0 M sodium chloride. The eluent was monitored by a UV-detector at 218 nm. A peak which eluted at around 17 min. was concentrated and desalted as above giving 15 mg of LSH-12. According to elemental analysis P was o.4% and S was 0.2%. Sugar analysis after hydrolysis, derivatization and GLC-MS showed glucosamine hydrochloride 10.5%, galactose 11.1% and xylose 2.0%. No fucose, mannosamine, galactosamine or anhydromannitol was found. NMR spectra indicate the presence of N-acetyl-glucosamine and glucuronic acid and the absence of iduronic acid as well as 2-0-sulfated iduronic acid. A FAB mass spectrum run in a negative mode showed the highest peaks at M/Z 1190 and 1176 and a base peak at M/Z 1074, indicating a hexasaccharide. The inhibition of endothelial cell proliferation was very strong both in the presence and absence of aFGF. For inhibition in the absence of aFGF, see Figure 14 and for inhibition in the presence of aFGF, see Figure 15.

Example 7

Heparan sulfate was prepared from a side fraction of the manufacturing of bovine lung heparin using ion exchange chromatography on Dowex 1-X2. Heparan sulfate was eluted with 1.5M sodium chloride. This heparan sulfate had elemental analysis C 25.3%, H 3.7%, N 2.2% and S 6.4%. the $SO_3^{2-}/CO_2^-$ ratio was 1.5 by conductimetric titrations. The optical rotation was [ $\alpha$ ]D$^{23}$ = 83.8° (c = 2 water). The anti-FXa was 6 IU/mg. No galactosamine was found by amino sugar analysis. This heparan sulfate was depolymerized as described for heparin in Example 1. The depolymerized heparan sulfate (0.5 g) was dissolved in sodium chloride (0.25M) and applied to Biogel P-6 (1800 x 50 mm). The column was eluted with 0.25M sodium chloride. Fractions corresponding to the peaks of the chromatogram in Figure 13 were collected and desalted on Biogel P-6DG and lyophilized. Fractions 2, 3 and 4 of the chromatogram in Figure 13 were analysed by high performance ion exchange chromatography on Mono Q (5/5) and eluted by a sodium chloride gradient as described in Example 6. The chromatograms of these fractions showed peaks having the same elution positions as those of the low sulfate oligosaccharides of the invention which were isolated from the corresponding fractions derived from heparin.

Biological Tests

The angiogenic response of the chorioallantoic membrane (CAM) to the novel low sulfate oligosaccharide containing fractions were investigated on fertilized chicken eggs. Non-incubated (0-day) fertilized eggs obtained from Linköpings Kontrollhönseri, Linköping, Sweden were stored in a low temperature incubator 13-17°C until ready for incubation at 37°C in an Egg Incubator (Andersson & Bonde TYP 40). Incubated eggs (37°C) were cracked on the 3rd day of incubation (3-day) and the entire contents were poured into a Petri dish. Only eggs with unbroken yolk were used for further incubation in a $CO_2$ tissue culture incubator with 3% $CO_2$ at 37°C with elevated relative humidity. After 3 days incubation (6-day) in $CO_2$ atmosphere the eggs are implanted with a methyl cellulose dish prepared as described below. Methyl cellulose was added to triple distilled water at a concentration of 0.5%, then autoclaved 30 minutes at 138 kPa and 120°C for sterility. This mixture contains a ball of gelled methyl cellulose. Subsequent slow agitation for 2-3 days at 5°C ensures complete dissolution. The solution was then kept in a refrigerator until ready for use. The test material was suspended in the methyl cellulose at room temperature to a final concentration of 1 to 50 mg/10 μl. Aliquots of 10 μl methyl cellulose containing the test sample were deposited onto the end of a Teflon (Trade Mark) rod (3 mm diam.). When the methyl cellulose test material disc is dry (30-40 min) it is lifted off the Teflon with 3 fine forceps and placed on the peripheral part of a 6-day CAM in order to avoid non-growing vessels. On day 8 the growing vessels under and around the transparent disc are examined under a dissecting microscope (x26 to x 40 magnification). The inhibition of vessel growth is scored either as no inhibition of vessel growth or as inhibiting vessel growth. Each compound was tested on a minimum of 15 eggs and the scoring was performed by two technicians who were uninformed concerning the nature and concentration of the test substance. The results are expressed as percent eggs showing inhibition.

Tissue culture.

Capillary Endothelial Cells (EC) were isolated and cultured from bovine adrenal cortices by the method of Folkman et al. (Proc. Natl. Acad. Sci. 1979, 76, 5217-5221). The cells were cultured in Dulbecco's modified Eagle's medium (DMEM) supplemented with 10% calf serum and 5 μl/ml of a crude retinal extract (Gitlin J D and D'Amore, Microvas. Res. 1983, 26, 74-80) in gelatin-coated tissue culture plates. The endothelial nature of the cells was assessed by staining with antisera to von Willebrand's factor and by the ability of the cells to take up fluoresceinated, acetylated, low density lipoprotein.

Proliferation Assays.

The ability of various heparin-derived oligosaccharides to influence EC growth in the presence or absence of purified aFGF was assessed using a capillary endothelial cell proliferation assay. The capillary endothelial cells were plated at 10,000 cells/well into gelatin-coated multi-well (2.1 cm²/well) dishes in DMEM with 5% calf serum and allowed to attach overnight. The following day the media were aspirated and replaced with fresh DMEM with 5% calf serum. For the assay of potentiation a sub-saturating dose of aFGF (1-5 3T3 units/ml), the equivalent of 0.2-1 ng/ml of aFGF, was added along with the heparin fragment to be tested at 10 μg/ml; for the assay of inhibition the heparin-derived oligosaccharides were added alone. aFGF was purified from bovine brain by the method of Lobb R R and Felt J W (Biochemistry 1984, 23, 6925-6929). Intact heparin and aFGF were added to separate cultures as controls. The cells were allowed to grow in the presence of the additions for three days, then trypsinized and counted using a Coulter Counter.

The assay was also performed in presence of larger amounts (2 ng/ml and 6 ng/ml) of a human aFGF prepared by recombinant-DNA technique. The larger amount (6 ng/ml) being a saturating dose of aFGF.

## Claims

1. A fraction derived from heparin or heparan sulfate, said fraction comprising low molecular weight oligosaccharides having a molecular weight between 1,000 and 2,000, characterized in that the majority of the low molecular weight oligosaccharides are low sulfate oligosaccharides having a sulfur content of less than 9 weight percent, whereby said fraction inhibits capillary endothelial cell proliferation or angiogenesis and has no significant anticoagulation activity.

2. A fraction as claimed in Claim 1, consisting essentially of said low sulfate oligosaccharides.

3. A fraction as claimed in Claim 1 or Claim 2, wherein said low sulfate oligosaccharides have a molecular weight between 1000 and 1800.

4. A fraction as claimed in Claim 3, wherein said low sulfate oligosaccharides have a molecular weight between 1000 and 1600.

5. A fraction as claimed in any one of the preceding claims, wherein said low sulfate oligosaccharides have a sulfur content of less than 7 weight percent.

6. A fraction as claimed in Claim 5, wherein said low sulfate oligosaccharides have a sulfur content of less than 4 weight percent.

7. A fraction as claimed in any one of the preceding claims, wherein said low sulfate oligosaccharides contain 6 to 8 saccharide units.

8. A fraction as claimed in any one of the preceding claims, wherein said low sulfate oligosaccharides comprise an uronic acid residue and a glucosamine residue.

9. A fraction as claimed in Claim 3, wherein said low sulfate oligosaccharides further comprise a galactose residue.

10. A fraction as claimed in Claim 9, wherein said low sulfate oligosaccharides further comprise a xylose residue.

11. A fraction as claimed in any one of the preceding claims which inhibits angiogenesis in chorioallantoic membranes when administered in an amount of between 1 μg and 50 μg.

12. A fraction as claimed in any one of the preceding claims for use in the inhibition of angiogenesis.

13. The use of a fraction as claimed in any one of Claims 1 to 11 in the manufacture of a medicament for the inhibition of angiogenesis.

14. A pharmaceutical composition comprising a fraction as claimed in any one of Claims 1 to 11 and a pharmaceutically acceptable carrier or diluent.

15. An essentially pure low molecular weight oligosaccharide having a molecular weight between 1,000 and 2,000 obtainable from heparin or heparan sulfate, characterized in that said oligosaccharide has a sulfur content of less than 9 weight percent, inhibits capillary endothelial cell proliferation or angiogenesis and has no significant anticoagulation activity.

16. An oligosaccharide as claimed in Claim 15 having a molecular weight, sulfur content and/or structure as defined in any one of Claims 3 to 10.

17. An oligosaccharide as claimed in Claim 15 or Claim 16 for use in the inhibition of angiogenesis.

18. The use of an oligosaccharide as claimed in Claim 15 or Claim 16 in the manufacture of a medicament for the inhibition of angiogenesis.

19. A pharmaceutical composition comprising an oligosaccharide as claimed in Claim 15 or Claim 16 and a pharmaceutically acceptable carrier or diluent.

Claims for the following Contracting States: GR, ES

1. A method for the preparation of a fraction comprising low molecular weight oligosaccharides having a molecular weight between 1,000 and 2,000, which comprises fractionating depolymerized heparin or heparan sulfate by size and charge to isolate a fraction in which the majority of the low molecular weight oligosaccharides are low sulfate oligosaccharides having a sulfur content of less than 9 weight percent, whereby said fraction inhibits capillary endothelial cell proliferation or angiogenesis and has no significant anticoagulation activity.

2. A method as claimed in Claim 1, wherein the fractionation is conducted to provide a fraction consisting essentially of said low sulfate oligosaccharides.

3. A method as claimed in Claim 1 or Claim 2, wherein the fractionation is conducted to provide a faction in which said low sulfate oligosaccharides have a molecular weight between 1000 and 1800.

4. A method as claimed in Claim 3, wherein the fractionation is conducted to provide a fraction in which said low sulfate oligosaccharides have a molecular weight between 1000 and 1600.

5. A method as claimed in any one of the preceding claims, wherein the fractionation is conducted to provide a fraction in which said low sulfate oligosaccharides have a sulfur content of less than 7 weight percent.

7. A method as claimed in any one of the preceding claims, wherein the fractionation is conducted to provide a fraction in which said low sulfate oligosaccharides contain 6 to 8 saccharide units.

8. A method as claimed in any one of the preceding claims, wherein the fractionation is conducted to provide a fraction in which said low sulfate oligosaccharides comprise an uronic acid residue and a glucosamine residue.

9. A method as claimed in Claim 8, wherein the fractionation is conducted to provide a fraction in which said low sulfate oligosaccharides further comprise a galactose residue.

10. A method as claimed in Claim 9, wherein the fractionation is conducted to provide a fraction in which said low sulfate oligosaccharides further comprise a xylose residue.

11. A method as claimed in any one of the preceding claims, wherein the fractionation is conducted to provide a fraction which inhibits angiogenesis in chorioallantoic membranes when administered in an amount of between 1 μg and 50 μg.

12. A method for the preparation of an essentially pure low molecular weight oligosaccharide having a molecular weight between 1,000 and 2,000 obtainable from heparin or heparan sulfate, which comprises fractionating depolymerized heparin or heparan sulfate by size and charge to isolate a fraction in which said oligosaccharide has a sulfur content of less than 9 weight percent, inhibits capillary endothelial cell proliferation or angiogenesis and has no significant anticoagulation activity.

13. A method as claimed in Claim 12, wherein the fractionation is conducted to provide a fraction in having a molecular weight, sulfur content and/or structure as defined in any one of Claims 3 to 10.

Neu eingereicht / Newly fil
Nouvellement déposé

FIG.1

INHIBITION OF ANGIOGENESIS

LOW SULPHATE HEXASACCHARIDE LSH-1

FIG. 2

## FIG.3

# FIG.4

Capillary EC number(% of control)

■ +FGF

- aFGF — 100
- Heparin — 221·6
- LSH-1 — 77·7
- LSH-4 — 59·8
- LSH-5 — 66·6
- LSH-6 — 73·6
- HSH-2 — 108·2

1 2 3 4 5 6 7

# FIG.5

Capillary EC number (% of control)

■ −FGF

- Water — 100
- Heparin — 85·7
- LSH-1 — 60·8
- LSH-4 — 34·4
- LSH-5 — 52·5
- LSH-6 — 53·9
- HSH-2 — 69·1

1 2 3 4 5 6 7

FIG.6
Low sulfated hexasaccharide
LSH-1

FIG.7
Low sulfated hexasaccharide
LSH-5

INHIBITION OF ANGIOGENESIS      Nouvellement déposé

## FIG.8

### LOW SULPHATE HEXASACCHARIDE LSH-10

## FIG.9

FIG.10

SALT

FIG.11

Low sulfated hexasaccharide
LSH-11

5.5      5.0      4.5      4.0      3.5      3.0      2.5      2.0   PPM

## FIG.12

## FIG.13

FIG.14

Capillary Endothelial Cell Number

(a) ■ .01 µg/ml
(b) ▦ .1 µg/ml
(c) ▨ 1.0 µg/ml
(d) ▦ 10 µg/ml
(e) ▨ water µg/ml
(f) ▨ heparin 10 µg/ml

LSH-11    LSH-12    Controls
Saccharides

FIG.15

Capillary Endothelial Cell Number

(a) ■ .01 µg/ml
(b) ▦ .1 µg/ml
(c) ▨ 1.0 µg/ml
(d) ▦ 10 µg/ml
(e) ▨ water
(f) ▨ aFGF(4ng/ml)
(g) ▤ aFGF(4ng/ml)+ heparin 10 µg/ml

LSH-11    LSH-12    Controls
Saccharides

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 110, no. 9, 27th February 1989, page 14, abstract no. 68982s, Columbus, Ohio, US; T.G. WRIGHT et al.: "Structural determinants of heparin's growth inhibitory activity. Interdependence of oligosaccharide size and charge", & J. BIOL. CHEM. 1989, 264(3), 1534-42 * Abstract * --- | 1-19 | A 61 K 31/725 C 08 B 37/10 |
| A | CHEMICAL ABSTRACTS, vol. 105, no. 5, 4th August 1986, page 20, abstract no. 35149u, Columbus, Ohio, US; J.J. CASTELLOT Jr., et al.: "Structural determinants of the capacity of heparin to inhibit the proliferation of vascular smooth muscle cells. II. evidence for a pentasaccharide sequence that contains a 3-O-sulfate group", & J. CELL. BIOL. 1986, 102(5), 1979-84 * Abstract * --- | 1 | |
| A | EP-A-0 244 298 (SANOFI) * Page 13, lines 24-30 * --- | 1 | TECHNICAL FIELDS SEARCHED (Int. Cl.5) A 61 K C 08 B |
| A | ANALYTICAL BIOCHEMISTRY, vol. 150, no. 2, 1st November 1985, pages 325-331, Academic Press, Inc., New York, US; K.G. RICE et al.: "High-performance liquid chromatograohic separation of heparin-derived oligosaccharides" * Figure 1 * --- | 1 | |
| A | FR-A-2 548 672 (PHARMUKA LABORATOIRES) * Abstract * --- -/- | | |

The present search report has been drawn up for all claims

| Place of search | Date of completion of the search | Examiner |
|---|---|---|
| THE HAGUE | 16-07-1990 | SOMERVILLE F.M. |

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | THE JOURNAL OF BIOLOGICAL CHEMISTRY, vol. 264, no. 12, 25th April 1989, pages 6892-6897, The American Society for Biochemistry and Molecular Biology, Inc, Baltimore, US; J. SUDHALTER et al.: "Importance of size, sulfation, and antocoagulant activity in the potentiation of acidic fibroblast growth factor by heparin" * Page 6894, column 2 * ----- | 1-19 | |

TECHNICAL FIELDS SEARCHED (Int. Cl.5)

The present search report has been drawn up for all claims

| Place of search | Date of completion of the search | Examiner |
|---|---|---|
| THE HAGUE | 16-07-1990 | SOMERVILLE F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)